# EUROPEAN PATENT APPLICATION

(11) **EP 4 574 072 A1**
(43) Date of publication of application: **25.06.2025**
(21) Application number: 24220437.8
(22) Date of filing: 17.12.2024
(51) Int. Cl.: A61B 18/14, A61B 18/00, A61B 34/20

(54) **ABLATION ELECTRODE ASSEMBLIES COMPRISING ENHANCED POSITION SENSING CAPABILITIES**

(30) Priority: 18.12.2023 US 202363611547 P; 16.07.2024 US 202418774741
(71) Applicant: Biosense Webster (Israel) Ltd., 2066717 Yokneam (IL)
(72) Inventor: GOVARI, Assaf, 2066717 Yokneam (IL); BEECKLER, Christopher Thomas, Irvine, CA 92618 (US)
(74) Representative: Carpmaels & Ransford LLP

(57) **Abstract**

The disclosed technology includes an electrode assembly comprising an electrode body comprising a top surface and a bottom surface. The electrode body can be configured to deliver ablative energy to tissue through at least the top surface. The electrode assembly can further include a coil disposed near the bottom surface. The coil can be configured to generate a voltage when subject to a magnetic field. The electrode assembly can include a member that is disposed near the coil and comprises a high-magnetic-permeability material.

## Description

### CROSS-REFERENCE TO RELATED APPLICATION

This application claims, under 35 U.S.C. § 119(e), priority to and the benefit of U.S. Provisional Patent Application No. 63/611,547, filed December 18, 2023 (Attorney Docket No.: BIO6838USPSP1 - 253757.000387), the entire contents of which are incorporated herein by reference.

### FIELD

The present invention relates generally to ablation electrode assemblies and more particularly to ablation electrode assemblies comprising electromagnetic coils and high-magnetic permeability materials to provide magnetic-based position sensing.

### BACKGROUND

Intravascular catheters are commonly used for mapping and ablation of myocardial tissue. Intravascular catheters typically include an end effector having one or more electrodes that are configured to receive electrical signals from the tissue for mapping and/or to deliver ablative energy to the tissue ablation. To ensure the electrodes are correctly positioned for mapping or ablation, some end effectors include position sensing such as electromagnetic position sensing or active current location (ACL) technology.

Electromagnetic position sensing typically utilizing electromagnetic coils disposed on or near the end effector that are configured to generate a current when subjected to a magnetic field induced by a magnetic field generator that is positioned externally to the patient. Many electromagnetic sensors, however, require coils having multiple windings to ensure the electromagnetic sensor has sufficient sensitivity to detect the electromagnetic field. Unfortunately, increasing the number of windings increases the size of the electromagnetic sensor. As will be appreciated, it is desirable for the end effector to be sufficiently small for cardiovascular procedures. Thus, there is a need in the art for methods of increasing the sensitivity of electromagnetic sensors while also reducing the size of the end effector. The technology disclosed herein addresses these issues.

### SUMMARY

There is provided, in accordance with an example of the present invention, an electrode assembly comprising an electrode body having a top surface and a bottom surface. The electrode body can be configured to deliver ablative energy to tissue through at least the top surface. The electrode assembly can further include a coil disposed near the bottom surface. The coil can be configured to generate a voltage when subject to a magnetic field. The electrode assembly can include a member that is disposed near the coil and comprises a high-magnetic-permeability material.

The disclosed technology can include a medical device comprising a flexible shaft extending along a longitudinal axis and a plurality of spines disposed at a distal end of the flexible shaft. The plurality of spines can be configured to bow radially outward from the longitudinal axis and to transition between an expanded configuration and a collapsed configuration. The medical device can further include a plurality of electrode assemblies attached to the plurality of spines. Each electrode assembly of the plurality of electrode assemblies can comprise an electrode body having a top surface and a bottom surface. The electrode body can be configured to deliver ablative energy to tissue through at least the top surface. The electrode assembly can include a coil disposed near the bottom surface. The coil can be configured to generate a voltage when subject to a magnetic field. The electrode assembly can include a member disposed near the coil and comprising a high-magnetic-permeability material.

Additional features, functionalities, and applications of the disclosed technology are discussed in more detail herein.

### BRIEF DESCRIPTION OF THE DRAWINGS

FIG. 1 is a schematic pictorial illustration of a medical system, in accordance with an example of the disclosed technology;
FIG. 2A is a schematic pictorial illustration of a basket catheter in an expanded configuration, in accordance with an example of the disclosed technology;
FIG. 2B is a schematic pictorial illustration of a basket catheter in a collapsed configuration in a sheath, in accordance with an example of the disclosed technology;
FIG. 3A is a perspective view of an electrode assembly, in accordance with an example of the disclosed technology;
FIG. 3B is a top perspective view of an electrode assembly, in accordance with an example of the disclosed technology;
FIG. 4A is a bottom view of an electrode assembly, in accordance with an example of the disclosed technology;
FIG. 4B is a section view of the electrode assembly of FIG. 4A taken along line A-A, in accordance with an example of the disclosed technology;
FIG. 4C is section view of an electrode assembly, in accordance with another example of the disclosed technology;
FIG. 4D is section view of an electrode assembly, in accordance with yet another example of the disclosed technology;
FIG. 4E is section view of an electrode assembly, in accordance with still another example of the disclosed technology;
FIG. 4F is section view of an electrode assembly, in accordance with another example of the disclosed technology;
FIG. 5A is a perspective view of an electrode, in accordance with another example of the disclosed technology;
FIG. 5B is a front view of the electrode of FIG. 5A, in accordance with an example of the disclosed technology;
FIG. 5C is a front view of an electrode, in accordance with yet another example of the disclosed technology;
FIG. 5D is a top perspective view of the electrode of FIG. 5C, in accordance with an example of the disclosed technology;
FIG. 5E is a perspective view of an electrode, in accordance with still another example of the disclosed technology; and
FIG. 5F is a front view of the electrode of FIG. 5E, in accordance with an example of the disclosed technology.

### DETAILED DESCRIPTION

The disclosed technology includes a plurality of electrode assemblies coupled to a plurality of spines forming a basket catheter disposed at a distal end of the flexible shaft. Each electrode assembly can include an electrode body designed to deliver ablative energy to tissue. Each electrode assembly can further comprise an electromagnetic coil attached to the electrode body that is configured to generate a current when subjected to an electromagnetic field that is generated by an electromagnetic field generator. Real time position of distal end of catheter may be tracked based on the current generated in the electromagnetic coils. By having the electromagnetic coil disposed on the electrode body, the overall size of the end effector can be reduced, and multiple electromagnetic coils can be utilized (for catheters having multiple electrode assemblies) to increase the accuracy of the position sensing. To further increase the sensitivity of the electromagnetic coils, the electrode assemblies can further include a high-magnetic permeability material disposed near the electromagnetic coil.

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as reversable or irreversible electroporation (IRE), referred throughout this disclosure interchangeably as pulsed electric field (PEF) and pulsed field ablation (PFA), or thermal energy such as radiofrequency (RF) ablation or cryoablation. Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer or inner surface without departing from the scope of the present disclosure.

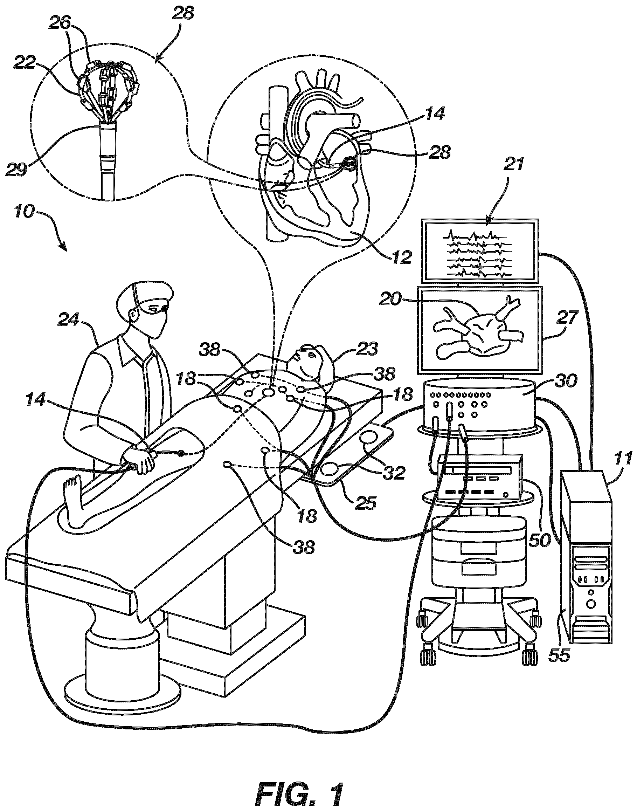

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. For sensing IEGM, Physician 24 brings a distal tip 28 of catheter 14 into contact with the heart wall for sensing a target site in heart 12. The end effector of the mapping catheter may include a basket catheter, a planar catheter, a focal catheter, a balloon catheter, etc. For ablation, physician 24 would similarly bring a distal end of an ablation catheter to a target site for ablating (as shown in inset of FIG. 1). Similarly, the end effector of the ablation catheter may include a basket catheter, a planar catheter, a focal catheter, a balloon catheter, etc.

Catheter 14 is an exemplary catheter that includes one and preferably multiple electrode assemblies 26 optionally distributed over a plurality of spines 22 at distal tip 28 and configured to deliver ablative energy to tissue. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, the entireties of each of which are incorporated herein by reference as if fully set forth herein.

System 10 includes one or more electrode patches 38 positioned for skin contact on patient 23 to establish location reference for location pad 25 as well as impedance-based tracking of electrode assemblies 26. For impedance-based tracking, electrical current is directed toward electrode assemblies 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, the entireties of each of which are incorporated herein by reference as if fully set forth herein.

A recorder 11 displays electrograms 21 captured with body surface ECG electrodes 18 and intracardiac electrograms (IEGM) captured with electrode assemblies 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

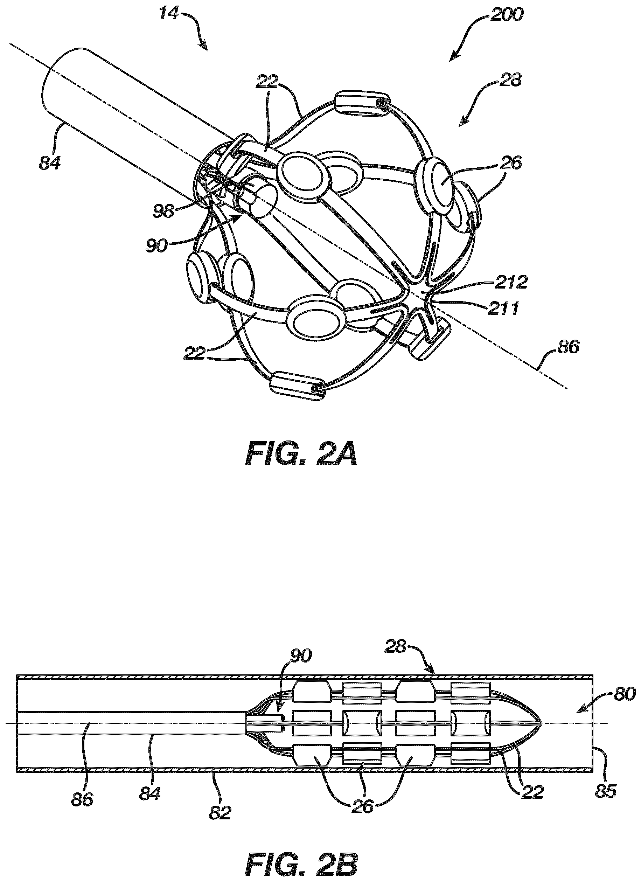

FIG. 2A is a schematic pictorial illustration showing a perspective view of a medical probe 200 having an end effector comprising a distal tip 28 that is a basket assembly in an expanded form when unconstrained, such as by being advanced out of a tubular shaft lumen 80 at a distal end 85 of a tubular shaft 82 (as depicted in FIG. 2B). FIG. 2B shows the basket assembly in a collapsed form within tubular shaft 82. In the expanded form (FIG. 2A), the spines 22 bow radially outward along a longitudinal axis 86 and in the collapsed form (FIG. 2B) the spines are constrained generally along the longitudinal axis 86 of tubular shaft 82 by an inner wall of the tubular shaft 82.

As shown in FIG. 2A, basket assembly 28 (referred to herein interchangeably as "distal tip") includes a plurality of flexible spines 22 that are formed at the end of a flexible shaft 84 and are connected at both ends. During a medical procedure, physician 24 can deploy basket assembly 28 by extending flexible shaft 84 from tubular shaft 82 causing the basket assembly 28 to exit the tubular shaft 82 and transition to the expanded form. Spines 22 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

In examples described herein, electrode assemblies 26 can be configured to deliver ablation energy (RF and/or IRE) to tissue in heart 12 or other parts of patient's 23 body. In addition to using electrode assemblies 26 to deliver ablation energy, the electrode assemblies can also be used to measure a physiological property such as local surface electrical potentials at respective locations on tissue in heart 12 (e.g., IEGM signals). The electrode assemblies 26 can be biased such that the larger surface area of the electrode body of the electrode assemblies 26 faces outwardly from the basket assembly 28 such that the electrode assemblies26 deliver a greater amount of electrical energy outwardly away from the basket assembly 28 (i.e., toward the heart 12 tissue) than inwardly toward the longitudinal axis 86 of the basket assembly 28.

Basket assembly 28 can include a stem 96 that extends longitudinally from a distal end 90 of the flexible shaft 84 towards distal end 212 of basket assembly 28. The disclosed technology can include an irrigation system that delivers irrigation fluid to spray ports 98. For example, stem 96 can include multiple spray ports 98, wherein each given spray port 98 can be angled to aim delivery of the irrigation fluid to either a given electrode assembly 26 or to tissue in heart 12. The electrode assemblies 26 can be cooled by aiming the irrigation fluid, via spray ports 98, at the portion of the electrode assemblies 26 on the inner side of the spines 22. The basket assembly 28 can include a central intersection 211 at a point where the spines 22 converge near the distal end 212.

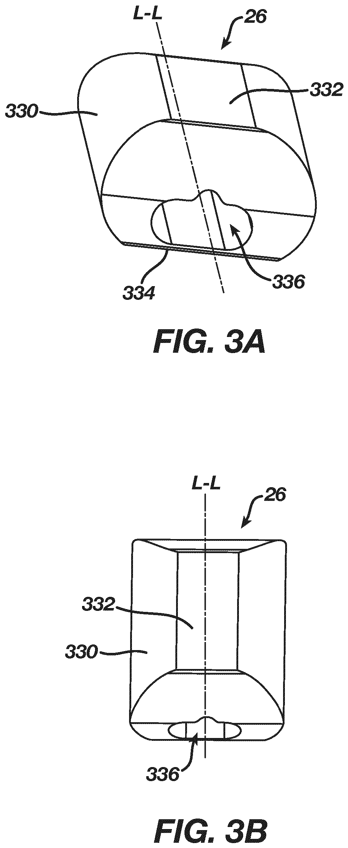

With reference to FIGs. 3A and 3B, an electrode assembly 26 can have an electrode body 330 having a top surface 332 and a bottom surface 334. The top surface 332 can be configured to face outwardly from the basket assembly 28 to contact tissue while the bottom surface 334 can be configured to face inwardly toward the longitudinal axis 86 of the basket assembly 28 such that the bottom surface 334 is unlikely to contact tissue. The electrode body 330 can be configured to deliver ablative energy to tissue through at least the top surface 332. The top surface 332 of the electrode body 330 can be substantially flat or planar. Alternatively, the top surface 332 can be rounded or have an undulating profile. The bottom surface 334 of the electrode body 330 can be substantially flat or planar. In some examples, a flat portion of the bottom surface 334 can comprise a larger surface area compared to the flat portion of the top surface 332.

The electrode body 330 can include a lumen 336 extending through the electrode body 330. The lumen 336 can be configured to receive a spine 22 of an end effector, such as a spine 22 of a basket assembly 28, as depicted in FIG. 2A. The spine 22 can be provided along a longitudinal axis L-L of the lumen 336. The electrode assemblies 26 can be fixed to the spine 22 of the basket assembly 28. Fixation of the electrode assemblies 26 to a spine 22 can comprise coupling between the spine 22 and an inner surface of the lumen 336. For example, the electrode assembly 26 can be crimped, glued, fastened, or otherwise coupled to the spine 22 to prevent the electrode assembly 26 from sliding along a length of the spine 22.

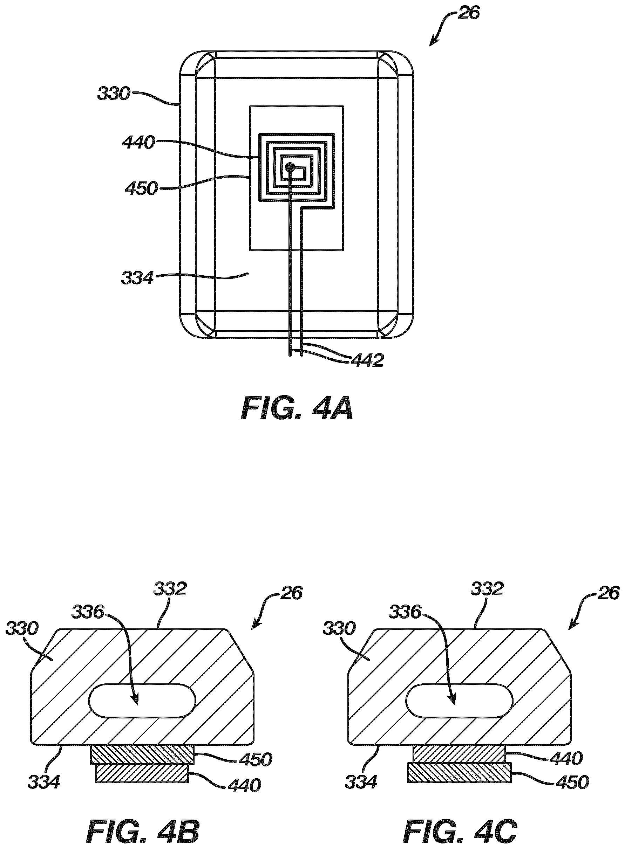

With reference to FIGs. 4A-4C, an electrode assembly 26 can comprise an electrode body 330, a coil 440 disposed near the bottom surface 334 of the electrode body 330, and a member 450, disposed near the bottom surface 334 of the electrode body 330. The electrode body 330 can be configured to deliver ablative energy to tissue through the top surface 332. The member 450 can comprise a high-magnetic-permeability material. The coil 440 can be configured to generate a current when subjected to a magnetic field. In some examples, the coil 440 can comprise a single axis sensor (SAS) or a triple axis sensor (TAS). The coil 400 can comprise a conductive material wound in a coil and disposed on the bottom surface 334 or a coil formed into a flexible circuit and the flexible circuit can be attached to the bottom surface 334. The coil 440 can comprise electrical leads 442 for conduction of current induced on the coil 440 to the patient interface unit 30. As will be appreciated, by attaching a coil 440 to the electrode body 330, it is possible to detect a position of each individual electrode of the basket assembly 28 (or at least each of the electrode assemblies 26 that include a coil 440). In this way, a physician 24 can more accurately determine a position of the electrode assemblies 26 before applying ablative energy to tissue.

The member 450 can be a high-magnetic-permeability material that is provided near the coil 440 to help increase the sensitivity of the coil 440. When the coil 440 comprises a single axis sensor, for example, the member 450 can help to enhance the electromagnetic field near the single axis coil. The high-magnetic permeability material can comprise a mu-metal, nanocrystalline metals, 99.95% pure iron, or a combination thereof. The member 450, for example, can comprise a relative magnetic permeability of approximately 50,000 to approximately 200,000. By placing the member 450 near the coil 440, the member 450 will provide a path for the magnetic field through the member 450 to the coil 440 to help increase the effect of the magnetic field on the coil 440, thereby increasing the sensitivity of the coil 440 in the magnetic field. The high-magnetic permeability properties of the member 450 provide a low reluctance path for magnetic flux. In this way, the disclosed technology can help to increase the magnetic field near the coil 440 to thereby induce a current on the coil 440 used for magnetic-based position sensing.

With reference to FIG. 4A, an electrode assembly 26 can comprise an electrode body 330 having a bottom surface 334. The electrode assembly 26 can comprise a coil 440 disposed near the bottom surface 334 of the electrode body 330. The electrode assembly 26 can comprise a member 450 that is also disposed near the bottom surface 334 of the electrode body 330. In some examples, the member 450 can be disposed on the bottom surface 334 of the electrode body 330 and the coil 440 can be disposed on a bottom surface of the member 450. Alternatively, the coil 440 can be disposed on the bottom surface 334 of the electrode body 330 and the member 450 can be disposed on the bottom surface of the coil 440.

FIG. 4B shows a section view of the electrode assembly 26 depicted in FIG. 4A taken along line A-A. The electrode assembly 26 can comprise an electrode body 330 having a top surface 332, a bottom surface 334, and a lumen 336 provided through the electrode body 330. As shown, the electrode assembly 26 can comprise a member 450 disposed on the bottom surface 334 of the electrode body 330 that is a high-magnetic-permeability material. A coil 440 can be disposed on a bottom side of the member 450, such that the member 450 is provided between the bottom surface 334 of the electrode body 330 and the coil 440.

With reference to FIG. 4C, in another example of the disclosed technology, the coil 440 can be disposed on the bottom surface 334 of the electrode body 330. Furthermore, the member 450 can be disposed on a bottom surface of the coil 440, such that the coil 440 is provided between the bottom surface 334 of the electrode body 330 and the member 450.

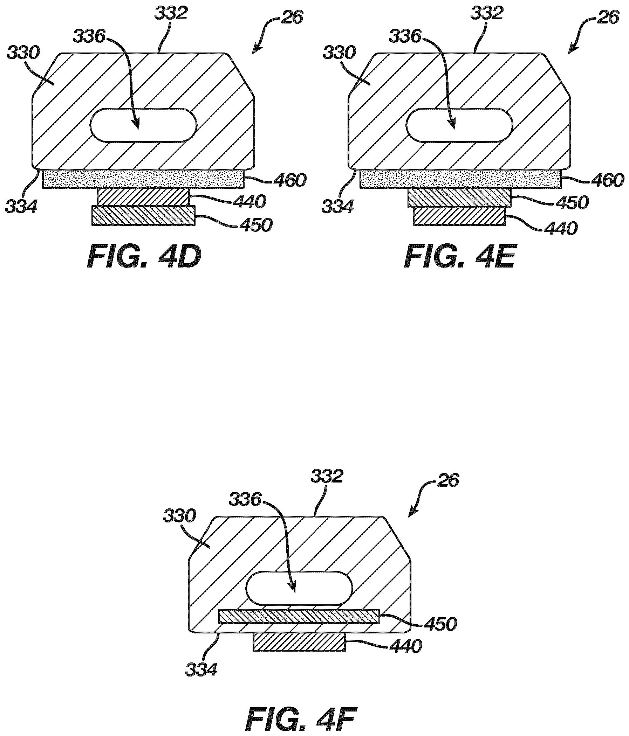

FIGs. 4D and 4E show a section view of an electrode assembly 26 comprising an insulative material 460 disposed on a bottom surface 334 of the electrode body 330. The insulative material 460 can be disposed on the bottom surface 334 of the electrode body 330 to form an insulative layer on the bottom surface 334 of the electrode body 330 to electrically insulate the electrode body 330 from the coil 440 and the member 450. In this manner, the insulative material 460 can prevent interference between the coil 440 and the electrode during an ablation and/or position location operation.

Turning to FIG. 4D the insulative material 460 can be disposed between the coil 440 and the bottom surface 334 of the electrode body 330. The member 450 can be disposed below the coil 440, such that the coil 440 is disposed between the insulative material 460 and the member 450. FIG. 4E, on the other hand, depicts an insulative material 460 disposed between the member 450 and the bottom surface 334 of the electrode body 330. The coil 440 can be disposed below the member 450, such that the member 450 is disposed between the insulative material 460 and the coil 440.

FIG. 4F depicts a section view of an electrode assembly 26 comprising a member 450 disposed within the electrode body 330. The electrode body 330 can be configured to deliver ablative energy to tissue through the top surface 332 The member 450 can comprise a high-magnetic-permeability material that is disposed at least partially within the electrode body 330. The member 450 can be disposed in the electrode body 330 near the bottom surface 334 of the electrode body 330 and coil 440 can be disposed on the bottom surface 334 of the electrode body 330. In this way, the member 450 can be disposed near the coil 440 and help to increase the sensitivity of the coil 440 when subject to a magnetic field as described herein.

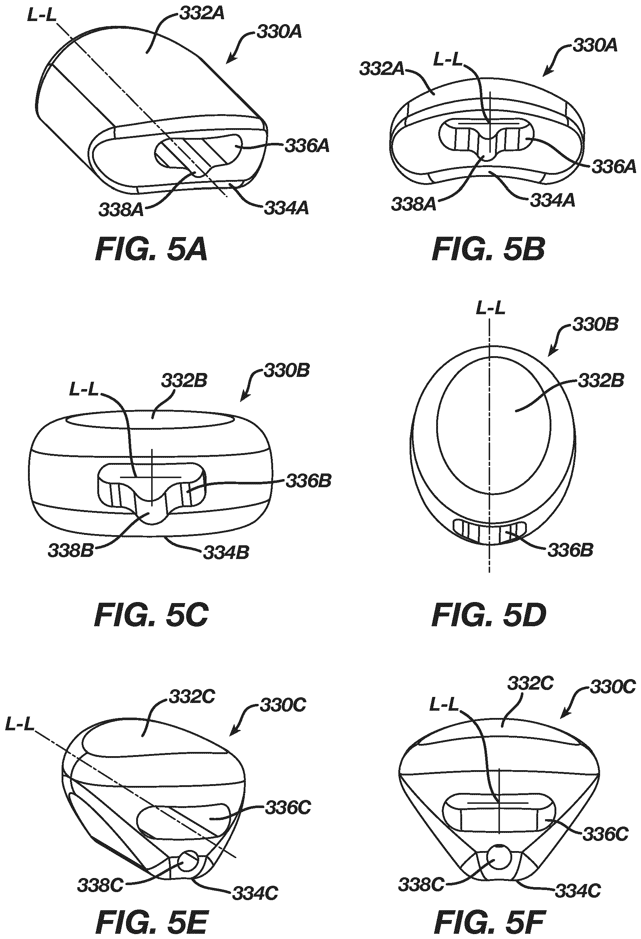

FIGs. 5A-5F depict variations of electrode bodies that can be used with the disclosed technology. That is, the features described herein above can be applicable to various other electrode body configurations, including, but not limited to, those electrode bodies shown in FIGs. 5A-5F. Furthermore, although each of the electrodes depicted in FIGs. 5A-5F include a lumen 336A, 336B, 336C configured to receive a spine (e.g., spine 22) for attaching the electrode body 330A, 330B, 330C to a spine, it will be appreciated that other types of attachment mechanisms are contemplated. For example, the disclosed technology can include electrode assemblies that are attached to spines by crimping, fastening, gluing, etc. the electrode assemblies to the spines rather than inserting a spine through the lumen 336A, 336B, 336C.

FIGs. 5A and 5B depict a variation of an electrode body 330A comprising a top surface 332A, a bottom surface 334A, and a lumen 336A configured to deliver ablative energy to tissue through at least the top surface 332A of the electrode body 330A. With reference to FIGs. 5A and 5B, electrode body 330A can be curved, such that the top surface 332A comprises a convex curvature while the bottom surface 334A comprises a convex curvature. The configuration can allow for an increased surface area at the top surface 332A, where ablative energy is applied to tissue, relative to the bottom surface 334A. The electrode body 330A can comprise a lumen 336A configured to receive a spine of a basket catheter therethrough and along a longitudinal axis L-L of the electrode body 330A. The electrode body 330A can further comprise a recess 338A to accommodate wiring which provides electricity for ablation. The recess 338A can be formed by an extended portion of the lumen 336A.

FIGs. 5C and 5D depict a variation of an electrode body 330B comprising a top surface 332B, a bottom surface 334B, and a lumen 336B configured to deliver ablative energy to tissue through at least the top surface 332B of the electrode body 330B. With reference to FIGs. 5C and 5D, electrode body 330B can be an elongated body comprising a substantially oval shape. The top surface 332B and the bottom surface 334B can be substantially flat. The electrode body 330B can comprise a lumen 336B configured to receive a spine of a basket catheter therethrough and along a longitudinal axis L-L of the electrode body 330B. The electrode body 330B can further comprise a recess 338B to accommodate wiring which provides electricity for ablation. The recess 338B can be formed by an extended portion of the lumen 336B.

FIGs. 5E and 5F depict a variation of an electrode body 330C comprising a top surface 332C, a bottom surface 334C, and a lumen 336C configured to deliver ablative energy to tissue through at least the top surface 332C of the electrode body 330C. With reference to FIGs. 5C and 5D, electrode body 330C can comprise a top surface 332C having a convex curvature. The electrode body 330C can taper from the top surface 332C toward the bottom surface 334C. The configuration can allow for an increased surface area at the top surface 332C, where ablative energy is applied to tissue, relative to the bottom surface 334C. The electrode body 330C can comprise a lumen 336C configured to receive a spine of a basket catheter therethrough and along a longitudinal axis L-L of the electrode body 330C. The electrode body 330B can further comprise an aperture 338C to accommodate wiring which provides electricity for ablation. The aperture 338C can be an additional through hole extending through the electrode body330C. The aperture 338C can run parallel to the lumen 336C.

Examples of materials ideally suited for forming the electrode bodies described herein include gold, platinum, and palladium (and their respective alloys). These materials also have high thermal conductivity which allows the minimal heat generated on the tissue (i.e., by the ablation energy delivered to the tissue) to be conducted through the electrode body to the bottom surfaces of the electrodes (i.e., the portions of the electrodes on the inner sides of the spines), and then to the blood pool in heart 12. Further, a conductive polymer material can be disposed along an outer surface of the electrode body.

The high-magnetic permeability material described herein, with respect to the present technology, can be made of any material, with materials of higher magnetic permeability being more suitable. Magnetic field lines preferentially travel through materials with high permeability. In various embodiments of the present technology, µ (Mu) -metals, amorphous metal alloys (also known as metallic glass alloys), nanocrystalline metals or 99.95% pure iron may be used. One particular branch of Mu metals and Metglas^{®} amorphous alloys (METGLAS is a registered trademark of Metglas, Inc. of Conway, South Carolina) are both particularly well suited for use with members of the present disclosure. As compared to air with a magnetic permeability equal to one (i.e., µ = 1), it has been found that Mu metals have a relative magnetic permeability of approximately 50,000 while 99.95% pure iron has a relative magnetic permeability of approximately 200,000.

The disclosed technology described herein can be further understood according to the following clauses:
Clause 1: An electrode assembly comprising: an electrode body comprising a top surface and a bottom surface, the electrode body being configured to deliver ablative energy to tissue through at least the top surface; a coil disposed near the bottom surface, the coil configured to generate a voltage when subject to a magnetic field; and a member disposed near the coil and comprising a high-magnetic-permeability material.
Clause 2: The electrode assembly of clause 1, the member being disposed between the coil and the electrode body, the member being attached to the bottom surface of the electrode body.
Clause 3: The electrode assembly of clause 2 further comprising an insulative material disposed between the member and the bottom surface of the electrode body.
Clause 4: The electrode assembly of clause 1, the coil being disposed between the member and the electrode body, the coil being attached to the bottom surface of the electrode body.
Clause 5: The electrode assembly of clause 4 further comprising an insulative material disposed between the coil and the bottom surface of the electrode body.
Clause 6: The electrode assembly of clause 1, the member being disposed at least partially in the electrode body.
Clause 7: The electrode assembly of any of the preceding clauses, the electrode body further comprising a lumen extending therethrough, the lumen configured to receive a spine of an end effector.
Clause 8: The electrode assembly of any of the preceding clauses, the coil comprising a single axis sensor (SAS).
Clause 9: The electrode assembly of any of the preceding clauses, the coil comprising a flexible circuit.
Clause 10: The electrode assembly of any of the preceding clauses, wherein the high-magnetic-permeability material comprises a Mu-metal.
Clause 11: The electrode assembly of any of the preceding clauses, further comprising a conductive polymer material disposed along an outer surface of the electrode body.
Clause 12: A medical device comprising: a flexible shaft extending along a longitudinal axis; a plurality of spines disposed at a distal end of the flexible shaft, the plurality of spines configured to bow radially outward from the longitudinal axis and to transition between an expanded configuration and a collapsed configuration; a plurality of electrode assemblies attached to the plurality of spines, each electrode assembly of the plurality of electrode assemblies comprising: an electrode body comprising a top surface and a bottom surface, the electrode body being configured to deliver ablative energy to tissue through at least the top surface; a coil disposed near the bottom surface, the coil configured to generate a voltage when subject to a magnetic field; and a member disposed near the coil and comprising a high-magnetic-permeability material.
Clause 13: The medical device of clause 12, the member being disposed between the coil and the electrode body, the member being attached to the bottom surface of the electrode body.
Clause 14: The medical device of clause 13 further comprising an insulative material disposed between the member and the bottom surface of the electrode body.
Clause 15: The medical device of clause 12, the coil being disposed between the member and the electrode body, the coil being attached to the bottom surface of the electrode body.
Clause 16: The medical device of clause 15 further comprising an insulative material disposed between the coil and the bottom surface of the electrode body.
Clause 17: The medical device of clause 12, the member being disposed at least partially in the electrode body.
Clause 18: The medical device of any of clauses 12-17, the electrode body further comprising a lumen extending therethrough, the lumen configured to receive a spine of an end effector.
Clause 19: The medical device of any of clauses 12-18, the coil comprising a flexible circuit.
Clause 20: The medical device of any of clauses 12-19, wherein the high-magnetic-permeability material comprises a Mu-metal.
Clause 21: The medical device of any of clauses 12-20, further comprising a conductive polymer material disposed along an outer surface of the electrode body.
Clause 22: The medical device of any of clauses 12-21, the coil comprising a single axis sensor (SAS).

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An electrode assembly comprising:
an electrode body comprising a top surface and a bottom surface, the electrode body being configured to deliver ablative energy to tissue through at least the top surface;
a coil disposed near the bottom surface, the coil configured to generate a voltage when subject to a magnetic field; and
a member disposed near the coil and comprising a high-magnetic-permeability material.

2. The electrode assembly of claim 1, the member being disposed between the coil and the electrode body, the member being attached to the bottom surface of the electrode body, optionally further comprising an insulative material disposed between the member and the bottom surface of the electrode body.

3. The electrode assembly of claim 1, the coil being disposed between the member and the electrode body, the coil being attached to the bottom surface of the electrode body, optionally further comprising an insulative material disposed between the coil and the bottom surface of the electrode body.

4. The electrode assembly of claim 1, the member being disposed at least partially in the electrode body.

5. The electrode assembly of any preceding claim, the electrode body further comprising a lumen extending therethrough, the lumen configured to receive a spine of an end effector.

6. The electrode assembly of any preceding claim, the coil comprising a single axis sensor (SAS) or a flexible circuit.

7. The electrode assembly of any preceding claim, wherein the high-magnetic-permeability material comprises a Mu-metal.

8. The electrode assembly of any preceding claim, further comprising a conductive polymer material disposed along an outer surface of the electrode body.

9. A medical device comprising:
a flexible shaft extending along a longitudinal axis;
a plurality of spines disposed at a distal end of the flexible shaft, the plurality of spines configured to bow radially outward from the longitudinal axis and to transition between an expanded configuration and a collapsed configuration;
a plurality of electrode assemblies attached to the plurality of spines, each electrode assembly of the plurality of electrode assemblies comprising:
an electrode body comprising a top surface and a bottom surface, the electrode body being configured to deliver ablative energy to tissue through at least the top surface;
a coil disposed near the bottom surface, the coil configured to generate a voltage when subject to a magnetic field; and
a member disposed near the coil and comprising a high-magnetic-permeability material.

10. The medical device of claim 9, the member being disposed between the coil and the electrode body, the member being attached to the bottom surface of the electrode body, optionally further comprising an insulative material disposed between the member and the bottom surface of the electrode body.

11. The medical device of claim 9, the coil being disposed between the member and the electrode body, the coil being attached to the bottom surface of the electrode body, optionally further comprising an insulative material disposed between the coil and the bottom surface of the electrode body.

12. The medical device of claim 9, the member being disposed at least partially in the electrode body.

13. The medical device of any of claims 9 to 12, the electrode body further comprising a lumen extending therethrough, the lumen configured to receive a spine of an end effector.

14. The medical device of any of claims 9 to 13, the coil comprising a flexible circuit.

15. The medical device of any of claims 9 to 14, wherein the high-magnetic-permeability material comprises a Mu-metal.
